# EUROPÄISCHE PATENTSCHRIFT

(11) **EP 0 818 206 B1**
(45) Veröffentlichungstag und Bekanntmachung des Hinweises auf die Patenterteilung: **09.10.2002**
(21) Anmeldenummer: 97111704.9
(22) Anmeldetag: 10.07.1997
(51) Int. Cl.: A61L 2/18, A61L 2/10, A61L 2/00

(54) **Verfahren zum Desinfizieren und Reinigen von Kleinteilen und dafür geeignete Vorrichtung**
Method and apparatus for cleaning and disinfecting small objects
Procédé et dispositif pour nettoyer et désinfecter de petits objets et dispositif

(30) Priorität: 12.07.1996 DE 19628133
(43) Veröffentlichungstag der Anmeldung: 14.01.1998
(73) Patentinhaber: Heraeus Noblelight GmbH, 63450 Hanau (DE)
(72) Erfinder: Roth, Angelika, Dr., 60439 Frankfurt (DE)
(74) Vertreter: Kühn, Hans-Christian

(56) Entgegenhaltungen:
- EP-A- 0 254 111
- EP-A- 0 518 450
- DE-A- 3 722 384
- DE-A- 4 242 170
- US-A- 5 120 499

## Beschreibung

Die Erfindung betrifft ein Verfahren zum Desinfizieren und Reinigen von Kleinteilen und Gerätschaften durch Behandlung in einer ein Oxidationsmittel enthaltenden Flüssigkeit und anschließendem Entfernen oder Eliminieren des Oxidationsmittels. Weiterhin betrifft die Erfindung eine Vorrichtung zur Durchführung des Verfahrens, mit einem Gehäuse, das einen Behandlungsraum zur Aufnahme einer Reinigungs-Flüssigkeit umschließt, .

Derartige Verfahren, beispielsweise zur Reinigung und Desinfektion von Kontaktlinsen unter Verwendung von Reinigungs-Lösungen, sowie die für die Reinigung und Desinfektion eingesetzten Vorrichtungen sind allgemein bekannt. Bei der Reinigungs-Lösung handelt es sich üblicherweise um eine wässrige peroxidhaltige Lösung, wobei der Peroxidgehalt auf etwa 3 Gew.-% eingestellt wird. Das Peroxid wirkt schwach oxidierend; es zerfällt in der Lösung jedoch in OH-Radikale, die ein starkes Oxidationsmittel darstellen und die daher reinigend und entkeimend wirken.

Üblicherweise werden für die Reinigung der Kontaktlinsen vorgefertigte Reinigungs-Lösungen verwendet. Diese wirken ätzend und sind daher gesundheitsschädlich. Die Kontaktlinsen werden in ein Kunststoffschälchen eingelegt, in das anschließend die Reinigungs-Lösung eingefüllt wird. Zur Vermeidung von Verätzungen sind die Kunststoffschälchen mit einem Schraubdeckel verschließbar.

Wegen seiner ätzenden Wirkung muß nach der Reinigung bzw. Desinfektion der Kontaktlinsen das überflüssige Oxidationsmittel entfernt werden. Dies kann durch Zugabe einer weiteren Chemikalie erfolgen, die beispielsweise eine vitaminhaltige Substanz enthalten kann. Die vitaminhaltige Substanz komplexiert die Oxidationsmittel der Reinigungs-Lösung und neutralisiert diese dadurch. Die Komplexbildung des Oxidationsmittels benötigt eine gewisse Zeit, wobei das Ende der Neutralisation nicht ohne weiteres erkennbar ist.

Bei den bekannten Verfahren ist die Zugabe geeigneter Chemikalien zur Reinigungs-Lösung., erforderlich. Sie sind daher problematisch hinsichtlich der Dosierung und der Entsorgung dieser Chemikalien. Die Reinigung und Desinfektion unter Verwendung der bekannten Reinigungs-Lösungen erfordert Sorgfalt und ein Maß ein Fachwissen, was den Einsatz für den allgemeinen Gebrauch erschwert. Darüberhinaus sind die bekannten Reinigungsmittel teuer.

Das Dokument US-A-5 120 499 beschreibt ein Verfahren zur Desinfektion von Kleinteilen und Gerätschaften durch Behandlung in einer ein Oxidationsmittel enthaltenden Flüssigkeit und anschließendem Entfernen des Oxidationsmittels, wobei das Oxidationsmittel, nämlich OH-Radikale, durch UV-Strahlung erzeugt wird.

Der vorliegenden Erfindung liegt die Aufgabe zugrunde, ein Verfahren zum Desinfizieren und Reinigen von Kleinteilen oder medizinischen Gerätschaften anzugeben, bei dem das Oxidationsmittel aus der Reinigungslösung eliminiert wird, und eine dafür geeignete Vorrichtung bereitzustellen.

Hinsichtlich des Verfahrens wird diese Aufgabe ausgehend von den eingangs genannten Verfahren erfindungsgemäß dadurch gelöst, daß das Oxidationsmittel durch Bestrahlen der Flüssigkeit mit UV-Strahlung einer ersten, kleineren Wellenlänge gebildet, und durch Bestrahlung mit UV-Strahlung einer zweiten, größeren Wellenlänge eliminiert wird.

Bei dem erfindungsgemäßen Verfahren ist die Zugabe von Oxidationsmitteln zur Flüssigkeit nicht erforderlich, wenn auch, als unterstützende Maßnahme, nicht ausgeschlossen. Die Flüssigkeit kann vor der Reinigungsbehandlung daher frei von Oxidationsmitteln und insoweit aus gesundheitlichen Gesichtspunkten unbedenklich sein. Die Oxidationsmittel werden einfach durch Bestrahlung der Flüssigkeit mit UV-Strahlung einer ersten, kleineren Wellenlänge gebildet. Damit die UV-Strahlung aus der Flüssigkeit Oxidationsmittel bilden kann, ist erforderlich, daß die Flüssigkeit geeignete chemische Ausgangsverbindungen enthält, die mit der kurzwelligen UV-Strahlung unter Bildung des Oxidationsmittels reagieren. Solche chemischen Ausgangsverbindungen können sowohl die Flüssigkeit selbst bilden oder sie können in der Flüssigkeit gelöst sein. Es ist aber nicht erforderlich, daß diese Ausgangsverbindungen selbst oxidierend wirken, sondern sie können aus gesundheitlich unbedenklichen Substanzen bestehen.

Auch für die Entfernung oder Neutralisation des Oxidationsmittels ist bei dem erfindungsgemäßen Verfahren keine Zugabe von Chemikalien erforderlich. Das Eliminieren des Oxidationsmittels erfolgt wiederum durch Bestrahlen der Reinigungs-Lösung mit UV-Strahlung einer zweiten, größeren Wellenlänge. Hierzu ist es erforderlich, daß das Oxidationsmittel selbst oder solche chemischen Verbindungen, die sich in der Flüssigkeit aus dem Oxidationsmittel im chemischen Gleichgewicht bilden, mit der längerwelligen UV-Strahlung unter Umsetzung in eine nicht oxidierend wirkende Substanz reagieren und dabei zerstört werden.

Zusätzlich ist natürlich darauf zu achten, daß bei der Bestrahlung mit UV-Strahlung der zweiten, längeren Wellenlänge nicht eine erneute Photolyse der chemischen Ausgangsverbindungen unter Erzeugung des Oxidationsmittels stattfindet.

Für den Einsatz des erfindungsgemäßen Reinigungs- und Desinfektionsverfahrens ist Fachwissen nicht erforderlich. Da die Bildung und Eliminierung des Oxidationsmittels lediglich auf der Wirkung von UV-Licht beruht, kann die Zudosierung von Chemikalien entfallen und damit einhergehende Fehler mit möglicherweise gesundheitlichen Folgeschäden sind insoweit ausgeschlossen. Zudem entfallen bei dem erfindungsgemäßen Verfahren Kosten für Reinigungsmittel oder die Kosten sind zumindest geringer als bei den bekannten Verfahren.

Bei einer bevorzugten Verfahrensweise werden aus einer wässrigen oder alkoholischen Reinigungs-Lösung OH-Radikale durch Bestrahlen mit UV-Strahlung einer Wellenlänge von unterhalb 190 nm gebildet und durch Bestrahlen mit UV-Strahlung einer Wellenlänge im Bereich von 190 nm bis 250 nm eliminiert.

Dabei wird beim Bestrahlen mit der kurzwelligen Strahlung das Oxidationsmittel, nämlich die OH-Radikale, aus H₂O-Molekülen oder aus Hydroxyl-Gruppen gebildet. Diese "Photolyse" von Wasser ist bei Bestrahlung mit UV-Strahlung mit Wellenlängen von unterhalb 190 nm zu beobachten. Die Zugabe von Chemikalien ist hierfür nicht erforderlich. Bei der Photolyse der wässrigen oder alkoholischen Reinigungs-Lösung bilden sich auch H-Radikale.

Die OH- bzw. H-Radikale werden nach der Reinigung bzw. der Desinfektion eliminiert durch Bestrahlung mit UV-Strahlung einer Wellenlänge im Bereich von 190 nm bis 250 nm. Da die Photolyse einer wässrigen oder alkoholischen Reinigungs-Lösung unter Bildung von OH-Radikalen aus H₂O-Molekülen erst bei Wellenlangen von unterhalb 190 nm einsetzt, wird für die Eliminierung der Radikale eine UV-Strahlung einer längeren Wellenlänge verwendet. In wässrigen oder alkoholischen Lösungen reagieren OH-Radikale miteinander unter Bildung von H₂O₂-Molekülen. H₂O₂-Moleküle zerfallen, wenn auch langsam bei Tageslicht, jedoch beschleunigt bei Bestrahlung mit UV-Licht. Aufgrund der Bestrahlung mit UV-Strahlung einer Wellenlänge im Bereich von 190 nm bis 250 nm rekombinieren die H₂O₂-Moleküle sowie die in der Reinigungs-Lösung vorhandenen Radikale sehr schnell zu Wasser-bzw. zu Alkohol-Molekülen.

Auch hierfür ist die Zugabe von Chemikalien also nicht erforderlich, wenn auch, als unterstützende Maßnahme, nicht ausgeschlossen.

Unter einer alkoholischen Reinigungslösung wird eine Lösung mit einem bei Raumtemperatur flüssigen Alkohol verstanden.

Als besonders vorteilhaft hat es sich erwiesen, zum Bilden des Oxidationsmittels UV-Strahlung einer Wellenlänge um 172 nm und zum Eliminieren des Oxidationsmittels UV-Strahlung einer Wellenlänge um 222 nm zu verwenden. Diese Wellenlängen der UV-Strahlung sind besonders gut auf die Bildung bzw. Eliminierung von OH-Radikalen aus wässrigen oder alkoholischen Lösungen abgestimmt.

Besonders einfach und betriebssicher gestaltet sich ein Verfahren, bei dem die Art und Dauer des Bestrahlens derart geregelt wird, daß nach einer vorgegebenen ersten Bestrahlungsdauer mit UV-Strahlung der ersten, kürzeren Wellenlänge, das Bestrahlen mit UV-Strahlung der zweiten, längeren Wellenlänge automatisch einsetzt. Der Verfahrensschritt der Eliminierung des Oxidationsmittels kann so nicht vergessen werden. Die Bedienungsfehler werden weiter dadurch vermieden, wenn auch die Bestrahlungsdauer mit der langwelligen UV-Strahlung für die Bedienungsperson fest vorgegeben wird. Die Bestrahlungsdauern hängen von der verwendeten UV-Strahlung, der Art der Reinigungsflüssigkeit und des Oxidationsmittels sowie der einzustellenden Konzentrationen ab. Sie sind anhand weniger Versuche nach den konkreten Erfordernissen optimierbar und können dann fest vorgegeben werden.

Besonders bewährt hat es sich, zur Erzeugung der UV-Strahlung sowohl der kürzeren als auch der längeren Wellenlänge Excimer-Strahler einzusetzen. Die von Excimer-Strahlern ausgehende UV-Strahlung ist nahezu monochrom. Wellenlängenbereiche im Infraroten sind darin nicht enthalten. Von daher wird eine Aufwärmung, wie sie beispielsweise bei Quecksilberstrahlem aufgrund des Infrarot-Anteils der Strahlung beobachtet wird, vermieden.

Hinsichtlich der Vorrichtung wird die oben genannte Aufgabe erfindungsgemäß dadurch gelöst, daß innerhalb des Gehäuses ein erster, einen ersten Entladungsraum aufweisender Excimer-Strahler zur Erzeugung von UV-Strahlung einer kürzeren Wellenlänge und ein zweiter, einen zweiten Entladungsraum aufweisender Excimer-Strahler zur Erzeugung von UV-Strahlung einer längeren Wellenlänge derart angeordnet sind, daß die von den Entladungsräumen ausgehende UV-Strahlung auf den Behandlungsraum einwirkt.

Ein geeigneter Excimerstrahler ist beispielsweise aus der EP-A1 254 111 bekannt. Die Merkmale des dort beschriebenen Excimerstrahlers werden hiermit in diese Anmeldung ausdrücklich einbezogen, soweit sie die Erzeugung der Excimer-Entladung betreffen. Dies gilt insbesondere für die Auswahl geeigneter Füllgase im Entladungsraum. Damit die von den Entladungsräumen ausgehende UV-Strahlung auf den Behandlungsraum einwirken kann, müssen die dem Behandlungsraum zugewandten Wandungen der Entladungsräume wenigstens teilweise für UV-Strahlung durchlässig sein.

Durch die Anordnung der beiden Excimer-Strahler innerhalb eines Gehäuses ergibt sich eine besonders einfache und leicht zu handhabende Vorrichtung.

In einer bevorzugten Ausführungsform weist der erste Entladungsraum eine Gasfüllung auf, die Xenon enthält und der zweite Entladungsraum eine Gasfüllung, die Krypton und Chlor enthält. Der erste Entladungsraum gibt beispielsweise UV-Strahlung einer Wellenlänge um 172 nm, der zweite Entladungsraum UV-Strahlung einer Wellenlänge um 222 nm ab. Derartige Excimer-Strahler sind handelsüblich, langlebig und betriebssicher.

Vorteilhafterweise grenzt der erste Entladungsraum an den Behandlungsraum unmittelbar an. Eine Ozonisierung der Luft innerhalb des Behandlungsraumes kann dadurch leicht vermieden werden. Hierzu ist der Behandlungsraum beispielsweise mit einer Flüssigkeit gefüllt, deren Füllstand so gewählt ist, daß die von dem ersten Entladungsräumen auf den Behandlungsraum einwirkende UV-Strahlung ausschließlich oder überwiegend auf die Flüssigkeit trifft.

Es hat sich insbesondere eine Ausführungsform der erfindungsgemäßen Vorrichtung bewährt, bei der der erste Entladungsraum und der zweite Entladungsraum in Form koaxial zueinander verlaufender Ringspalte ausgebildet sind, die eine gemeinsame äußere Elektrode und eine gemeinsame innere Elektrode aufweisend, den Behandlungsraum seitlich mindestens teilweise umschließen. Die Entladungsräume werden bei dieser Ausführungsform, die sich durch einen besonders einfachen konstruktiven Aufbau auszeichnet, durch zwei koaxiale Rohre gebildet, wobei die Stirnseiten jeweils verschlossen sind. Diese "Doppelrohr" bildet die seitliche Begrenzungswand des Behandlungsraumes. Um die Ozonisierung der Luft zu verringern ist vorteilhafterweise ist derjenige Entladungsraum, der die UV-Strahlung mit der kürzeren Wellenlänge abgibt, innen angeordnet.

Ausführungsformen der Erfindung sind in der Zeichnung dargestellt und werden nachfolgend näher erläutert. In der Zeichnung zeigen im einzelnen in schematischer Darstellung
- **Figur 1:**: ein Behandlungsgefäß zur Reinigung von Kontaktlinsen in einem Längsschnitt und
- **Figur 2:**: eine weitere Ausführungsform eines erfindungsgemäßen Behandlungsgefäßes zur Desinfektion von medizinischem Gerät in einem Längsschnitt.

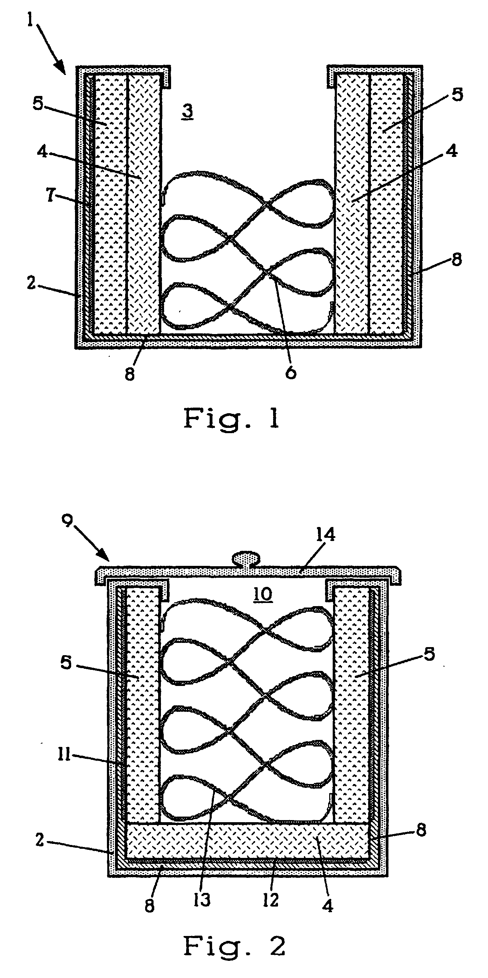

In der **Figur 1** ist die Bezugsziffer 1 insgesamt einem Reinigungsgefäß für die Reinigung und Desinfektion von Kontaktlinsen zugeordnet. Das Reinigungsgefäß 1 besteht aus einem metallischen Gehäuse 2, das einen Behandlungsraum 3 zur Aufnahme der Kontaktlinsen umgibt. In dem Gehäuse 2 ist ein Excimer-Strahler angeordnet, dessen Entladungsraum 4 unmittelbar an den Behandlungsraum 3 angrenzt. Die Wandungen des Entladungsraumes 4 bestehen aus Quarzglas. Der Entladungsraum 4 enthält eine Gasfüllung aus Krypton und Chlor und gibt UV-Strahlung einer Wellenlänge um 172 nm ab.

Innerhalb des Gehäuses 2 ist ein weiterer Excimer-Strahler angeordnet, dessen Entladungsraum mit der Bezugsziffer 5 bezeichnet ist und der eine Xenon-haltige Gasfüllung aufweist und UV-Strahlung einer Wellenlänge um 222 nm abgibt. Auch die Wandungen des Entladungsraumes 5 bestehen aus Quarzglas. Die Entladungsräume 4; 5 sind in Form koaxial zueinander angeordneter Ringspalte ausgebildet, die unmittelbar aneinander grenzen und die den Behandlungsraum 3 seitlich umschließen

Innerhalb des Behandlungsraumes 3 befindet sich eine Innenelektrode 6, die an der Innenwandung des Entladungsraumes 4 spiralig anliegt. Die Innenelektrode 6 erstreckt sich vom Boden bis zur etwa der halben Höhe des Behandlungsraumes 3. Die Höhe der Innenelektrode 6 bestimmt hier den Bereich der Entladung in den Entladungsräumen 4; 5. Im Ausführungsbeispiel brennt die stille elektrische Entladung nur im Bereich der Innenelektrode 6.

An der Außenseite des Entladungsraumes 5 ist eine metallische Schicht aufgebracht, die als Außenelektrode 7 wirkt. Die Innenelektrode 6 und die Außenelektrode 7 liegen an hochfrequenter Wechselspannung an. Die elektrischen Anschlüsse und die Spannungsquelle sind ist in der Figur 1 nicht dargestellt. Beide Elektroden 6; 7 sind den Excimer-Strahlern, bzw. den jeweiligen Entladungsräumen 4; 5 gemeinsam.

Zwischen der Innenseite des Gehäuses 2 und der Außenseite des Entladungsraumes 5 ist eine Isolationsschicht 8 aus einem elektrisch nicht leitenden Kunststoff vorgesehen. Aus Gründen des Strahlenschutzes ist das Gehäuse 2 mit einem aufklappbaren Deckel versehen, der in der Figur nicht dargestellt ist.

Nachfolgend wird das erfindungsgemäße Verfahren anhand der in Figur 1 dargestellten Ausführungsform des Reinigungsgefäßes näher erläutert.

Der Behandlungsraum 3 wird mit einer Reinigungsflüssigkeit, nämlich Wasser, gefüllt. Um eine Ozonisierung der Luft innerhalb des Behandlungsraumes 3 durch UV-Strahlung zu vermeiden, ist es sinnvoll, den Füllstand innerhalb des Behandlungsraumes 3 so zu wählen, daß die Innenelektrode 6, in deren Bereich die stille elektrische Entladung brennt, vollständig von Wasser umgeben ist.

Nach dem Einlegen der Kontaktlinsen in die Reinigungsflüssigkeit und dem Schließen des Deckels wird zunächst der dem Entladungsraum 4 zugeordnete Excimer-Strahler automatisch eingeschaltet. Dieser gibt UV-Strahlung einer Wellenlänge um 172 nm in den Behandlungsraum 3 ab. Dabei wird das Wasser unter Bildung oxidierend wirkender OH-Radikale photolysiert. Die oxidative Wirkung der OH-Radikale bewirkt eine Reinigung und Desinfektion der Kontaktlinsen.

Nach einer Bestrahlungszeit von ca. 5 Minuten wird der dem Entladungsraum 4 zuzuordnende Excimer-Strahler automatisch ab- und der dem Entladungsraum 5 zuzuordnende Excimer-Strahler automatisch eingeschaltet. Letzterer gibt UV-Strahlung einer Wellenlänge von 222 nm ab. Diese UV-Strahlung bewirkt keine weitere Photolyse des Wassers mehr. Andererseits wird sie von den aus jeweils zwei OH-Radikalen gebildeten Peroxid-Molekülen absorbiert. Diese werden in H₂O-Moleküle umgesetzt und dadurch aus dem chemischen Gleichgewicht laufend entfernt. Weiterhin rekombinieren auch OH- und H-Radikale unter Bildung von Wasser, so daß nach einer Bestrahlungsdauer von ca. 5 Minuten keine Radikale mehr in der Reinigungslösung enthalten sind. Der dem Entladungsraum 5 zuzuordnende Excimer-Strahler schaltet sich danach automatisch ab und die Kontaktlinsen können entnommen werden.

Sofern in Figur 2 die gleichen Bezugsziffern wie in Figur 1 verwendet sind, betreffen diese gleiche oder äquivalente Bauteile, wie sie anhand Figur 1 erläutert sind.

In **Figur 2** ist die Bezugsziffer 9 einem Desinfektionsgefäß für medizinische Kleinteile zugeordnet. Das Desinfektionsgefäß 9 besteht aus einem Gehäuse 2, das einen Desinfektionsraum 10 umschließt. Im Desinfektionsraum 10, der von oben gesehen einen kreisförmigen Querschnitt aufweist, werden medizinische Kleinteile desinfiziert.

Die seitlichen Wandungen des Desinfektionsraumes 10 bestehen aus Quarzglas und werden vom Entladungsraum 5 eines Excimer-Strahlers gebildet, der UV-Strahlung einer Wellenlänge von 222 nm abgibt. Der Boden des Desinfektionsraumes 10 besteht ebenfalls aus Quarzglas und wird von einem Entladungsraum 4 eines Excimer-Strahlers gebildet, der UV-Strahlung einer Wellenlänge von 172 nm abgibt.

An den dem Desinfektionsraum 10 abgewandten Oberflächen ist der Entladungsraum 5 mit einer Metallschicht 11 und der Entladungsraum 4 mit einer Metallschicht 12 versehen. Die Metallschichten 11; 12 wirken jeweils als Außenelektrode der Excimerstrahler. Die Innenelektrode für beide Excimer-Strahler bzw. für die Entladungsräume 4; 5 befindet sich innerhalb des Desinfektionsraumes 10 und ist in Form einer an den Innenwandungen des Desinfektionsraumes 10 anliegenden Spirale 13 ausgebildet. Die Spirale 13 ist somit eine den beiden Excimer-Strahlern bzw. beiden Entladungsräumen 4; 5 gemeinsame Innenelektrode. Sie reicht fast über die gesamte Höhe des Desinfektionsraumes 10. Die stille elektrische Entladung innerhalb des Entladungsraumes 5 brennt daher bei dieser Ausführungsform des Reinigungsgefäßes über die gesamte Höhe des Desinfektionsraumes 10.

Der Desinfektionsraum 10 ist mit einem abnehmbaren Deckel 14 verschließbar.

Die Desinfektion medizinischer Kleinteile unter Verwendung der in Figur 2 dargestellten Vorrichtung erfolgt in ähnlicher Weise, wie anhand der Figur 1 die Reinigung und Desinfektion von Kontaktlinsen erläutert worden ist. Ein Zusatz von oxidierend wirkenden Reinigungsmitteln oder Vitaminkomplexen zur Zerstörung dieser Mittel ist bei dem erfindungsgemäßen Verfahren nicht erforderlich.

## Patentansprüche

1. Verfahren zum Desinfizieren und Reinigen von Kleinteilen und Gerätschaften durch Behandlung in einer ein Oxidationsmittel enthaltenden Flüssigkeit und anschließendem Entfernen oder Eliminieren des Oxidationsmittels, **dadurch gekennzeichnet, daß** das Oxidationsmittel durch Bestrahlen der Flüssigkeit mit UV-Strahlung einer ersten, kleineren Wellenlänge gebildet, und durch Bestrahlung mit UV-Strahlung einer zweiten, größeren Wellenlänge eliminiert wird.

2. Verfahren nach Anspruch 1, **dadurch gekennzeichnet, daß** aus einer wässrigen oder alkoholischen Reinigungs-Lösung OH-Radikale durch Bestrahlen mit UV-Strahlung einer Wellenlänge von unterhalb 190 nm gebildet und durch Bestrahlen mit UV-Strahlung einer Wellenlänge im Bereich von 190 nm bis 250 nm eliminiert werden.

3. Verfahren nach Anspruch 2, **dadurch gekennzeichnet, daß** zum Bilden der OH-Radikale UV-Strahlung einer Wellenlänge um 172 nm und zum Eliminieren der OH-Radikale UV-Strahlung einer Wellenlänge um 222 nm verwendet wird.

4. Verfahren nach einem der vorhergehenden Ansprüche, **dadurch gekennzeichnet, daß** die Art und Dauer des Bestrahlens derart geregelt wird, **daß** nach einer vorgegebenen ersten Bestrahlungsdauer mit UV-Strahlung der ersten, kürzeren Wellenlänge, das Bestrahlen mit UV-Strahlung der zweiten, längeren Wellenlänge automatisch einsetzt.

5. Verfahren nach einem der vorhergehenden Ansprüche, **dadurch gekennzeichnet, daß** zur Erzeugung der UV-Strahlung sowohl der kürzeren als auch der längeren Wellenlänge ein Excimer-Strahler eingesetzt wird.

6. Vorrichtung zur Durchführung des Verfahrens nach einem der vorhergehenden Ansprüche, mit einem Gehäuse, das einen Behandlungsraum zur Aufnahme einer Reinigungs-Flüssigkeit umschließt, **dadurch gekennzeichnet, daß** innerhalb des Gehäuses (2) ein erster, einen ersten Entladungsraum (4) aufweisender Excimer-Strahler zur Erzeugung von UV-Strahlung einer kürzeren Wellenlänge und ein zweiter, einen zweiten Entladungsraum (5) aufweisender Excimer-Strahler zur Erzeugung von UV-Strahlung einer längeren Wellenlänge derart angeordnet sind, **daß** die von den Entladungsräumen (4;5) ausgehende UV-Strahlung auf den Behandlungsraum (3) einwirkt.

7. Vorrichtung nach Anspruch 6, **dadurch gekennzeichnet**, **dadurch gekennzeichnet, daß** der erste Entladungsraum (4) eine Gasfüllung, die Xenon enthält und der zweite Entladungsraum (5) eine Gasfüllung, die Krypton und Chlor enthält, aufweist.

8. Vorrichtung nach Anspruch 6 oder 7, **dadurch gekennzeichnet, daß** der erste Entladungsraum (4) an den Behandlungsraum (3) unmittelbar angrenzt.

9. Vorrichtung nach einem der Ansprüche 6. bis 8, **dadurch gekennzeichnet, daß** der erste Entladungsraum (4) und der zweite Entladungsraum (5) in Form koaxial zueinander verlaufender Ringspalte ausgebildet sind, die eine gemeinsame äußere Elektrode (7) und eine gemeinsame innere Elektrode (6) aufweisend, den Behandlungsraum (3) seitlich mindestens teilweise umschließen.

## Claims

1. Method for disinfecting and cleaning small parts and equipment by treatment in a liquid containing an oxidizing agent and subsequent removal or elimination of the oxidizing agent, **characterized in that** the oxidizing agent is formed by exposure of the liquid to UV radiation of a first, shorter wavelength and is eliminated by exposure to UV radiation of a second, longer wavelength.

2. Method according to Claim 1, **characterized in that** OH radicals are formed from an aqueous or alcoholic cleaning solution by exposure to UV radiation of a wavelength below 190 nm and are eliminated by exposure to UV radiation of a wavelength in the range from 190 nm to 250 nm.

3. Method according to Claim 2, **characterized in that** UV radiation of a wavelength of about 172 nm is used for the formation of the OH radicals and UV radiation of a wavelength of about 222 nm is used for the elimination of the OH radicals.

4. Method according to any of the preceding Claims, **characterized in that** the type and duration of the irradiation are regulated so **that** the exposure to UV radiation of the second, longer wavelength automatically starts after a predetermined first period of exposure to UV radiation of the first, shorter wavelength.

5. Method according to any of the preceding Claims, **characterized in that** an excimer lamp is used for producing the UV radiation, both of the shorter and of the longer wavelength.

6. Apparatus for carrying out the method according to any of the preceding Claims, comprising a housing which encloses a treatment space for receiving a cleaning liquid, **characterized in that** a first excimer lamp having a first discharge space (4) and intended for producing UV radiation of a shorter wavelength and a second excimer lamp having a second discharge space (5) and intended for producing UV radiation of a longer wavelength are arranged inside the housing (2) in such a way **that** the UV radiation emerging from the discharge spaces (4; 5) acts on the treatment space (3).

7. Apparatus according to Claim 6, **characterized in that** the first discharge space (4) has a gas filling which contains xenon and the second discharge space (5) has a gas filling which contains krypton and chlorine.

8. Apparatus according to Claim 6 or 7, **characterized in that** the first discharge space (4) is directly adjacent to the treatment space (3).

9. Apparatus according to any of Claims 6 to 8, **characterized in that** the first discharge space (4) and the second discharge space (5) are in the form of annular gaps which are coaxial with one another, have a common outer electrode (7) and a common inner electrode (6) and at least partly enclose the treatment space (3) laterally.

## Revendications

1. Procédé de désinfection et de nettoyage de petites pièces et d'appareillages par traitement dans un liquide contenant un oxydant et ensuite par enlèvement ou élimination de l'oxydant, **caractérisé en ce que** l'oxydant est formé par irradiation du liquide avec des rayons ultraviolets d'une première longueur d'ondes plus petite et éliminé par irradiation aux rayons ultraviolets d'une deuxième longueur d'ondes plus grande.

2. Procédé selon la revendication 1, **caractérisé en ce qu'**à partir d'une solution de nettoyage aqueuse ou alcoolisée, des radicaux OH sont formés par irradiation aux rayons ultraviolets d'une longueur d'ondes inférieure à 190 nm et sont éliminés par irradiation aux rayons ultraviolets d'une longueur d'ondes dans la plage de 190 nm à 250 nm.

3. Procédé selon la revendication 2, **caractérisé en ce que** pour former les radicaux OH, on utilise des rayons ultraviolets d'une longueur d'ondes d'environ 172 nm et pour éliminer les radicaux OH, on utilise des rayons ultraviolets d'une longueur d'ondes d'environ 222 nm.

4. Procédé selon l'une des revendications précédentes, **caractérisé en ce que** le type et la durée de l'irradiation sont réglés de telle sorte qu'après une première durée d'irradiation prédéterminée avec des rayons ultraviolets de la première longueur d'ondes plus courte, l'irradiation avec des rayons ultraviolets de la deuxième longueur d'ondes plus longue se déclenche automatiquement.

5. Procédé selon l'une des revendications précédentes, **caractérisé en ce qu'**on met en oeuvre une lampe d'irradiation à excimer pour engendrer les rayons ultraviolets de longueur d'ondes tant plus petite que plus grande.

6. Dispositif pour la mise en oeuvre du procédé selon l'une des revendications précédentes, comportant un boîtier qui entoure une chambre de traitement destinée à recevoir un liquide de nettoyage, **caractérisé en ce qu'**à l'intérieur du boîtier (2) sont agencées une première lampe d'irradiation à excimer présentant une première chambre de décharge (4) pour engendrer des rayons ultraviolets de longueur d'ondes plus courte et une deuxième lampe d'irradiation à excimer présentant une deuxième chambre de décharge (5) pour engendrer des rayons ultraviolets de longueur d'ondes plus longue, de telle sorte que les rayons ultraviolets émanant des chambres de décharge (4 ; 5) agissent sur la chambre de traitement (3).

7. Dispositif selon la revendication 6, **caractérisé en ce que** la première chambre de décharge (4) présente un remplissage de gaz qui contient du xénon et **en ce que** la deuxième chambre de décharge (5) présente un remplissage de gaz qui contient du krypton et du chlore.

8. Dispositif selon l'une ou l'autre des revendications 6 et 7, **caractérisé en ce que** la première chambre de décharge (4) est directement adjacente à la chambre de traitement (3).

9. Dispositif selon l'une des revendications 6 à 8, **caractérisé en ce que** la première chambre de décharge (4) et la deuxième chambre de décharge (5) sont réalisées sous forme de fentes annulaires s'étendant coaxialement l'une par rapport à l'autre qui, présentant une électrode extérieure (7) commune et une électrode intérieure (6) commune, entourent latéralement au moins partiellement la chambre de traitement (3).
